# FASCICULE DE BREVET EUROPEEN

(11) **EP 3 164 698 B1**
(45) Date de publication et mention de la délivrance du brevet: **13.03.2019**
(21) Numéro de dépôt: 15756187.9
(22) Date de dépôt: 30.06.2015
(51) Int. Cl.: G01N 21/03, G01N 21/11, G01N 21/3563, G01N 21/359, G01N 21/85, G01N 33/10, G01J 3/10, G01J 3/02, B07C 5/342, A01D 41/127, G01N 21/84

(54) **AGENCEMENT DE MESURE SPECTROMÉTRIQUE DE PRODUITS, TELS QUE DES CÉRÉALES, OLÉAGINEUX OU PRODUITS DÉRIVÉS**
ANORDNUNG ZUR SPEKTROMETRISCHEN MESSUNG VON PRODUKTEN WIE GETREIDE, ÖLHALTIGEN PRODUKTEN ODER DERIVATEN
ARRANGEMENT FOR THE SPECTROMETRIC MEASUREMENT OF PRODUCTS SUCH AS CEREALS, OLEAGINOUS PRODUCTS OR DERIVED PRODUCTS

(30) Priorité: 01.07.2014 FR 1401552
(43) Date de publication de la demande: 10.05.2017
(73) Titulaire: CHAUVIN ARNOUX, 75018 Paris (FR)
(72) Inventeur: LOPEZ, Eric, F-31600 Gaillac (FR); HURET, Vincent, F-31290 Villenouvelle (FR)
(74) Mandataire: Berger, Helmut
(86) Numéro de dépôt international: PCT/FR2015/051792
(87) Numéro de publication internationale: WO 2016/001572

(56) Documents cités:
- EP-A1- 1 707 945
- EP-A1- 2 236 909
- DE-B- 1 274 821
- US-A- 3 242 739
- US-A- 4 519 020
- US-A- 4 533 984
- US-A- 6 020 588
- US-A1- 2014 111 792

## Description

La présente invention concerne un agencement de mesure spectrométrique de céréales, oléagineux ou produits dérivés.

Il est connu d'analyser des échantillons de céréales et oléagineux par mesures spectrométriques en mode proche infrarouge, en vue par exemple de connaître leur pourcentage d'humidité, de protéine ou d'huile.

A cet effet, l'échantillon est analysé à l'état stationnaire à l'aide d'un rayonnement d'analyse en recevant un rayonnement d'analyse stabilisé et collimaté qui est émis par un émetteur 100 disposé d'un côté d'une chambre de lecture 300, avant d'être reçu ensuite, via un système optique de diffraction 400, par un récepteur 500 situé de l'autre côté (Fig. 1). EP 1 707 945 A1 décrit un agencement similaire.

Les contraintes d'un dispositif de mesure mobile spectroscopique d'échantillons résident dans la nécessité d'une parfaite stabilité mécanique de la source lumineuse qui ne doit pas dévier lors des vibrations éventuelles et dans l'écoulement séquentiel d'un échantillon de volume constant dans la chambre de lecture assujettie aux mêmes vibrations. Un écoulement régulier de l'échantillon permet une homogénéité régulière des céréales. En fonctionnement mobile de tels dispositifs, il est difficile de remédier au manque d'homogénéité naturel de l'échantillon de céréales ou oléagineux en vibration. Les grains qui composent l'échantillon sont légers et différent par leurs tailles. Lors de son transport dans un mécanisme complexe non adapté aux contraintes de vibrations, l'échantillon a tendance à se compacter, ne plus obéir aux lois de la gravité par enchevêtrement des graines en une masse compacte mis en pression par le poids des graines en attente par-dessus elles. Qui plus est, les variétés de céréales sont différentes par leurs tailles, allant d'un millimètre de diamètre pour le colza à plusieurs millimètres pour le maïs. Gérer mécaniquement cette diversité de taille en mouvement par un seul système mécanique s'avère donc difficile.

Un autre problème est de disposer d'une lumière correctement alignée et ayant des rayons parallèles pour l'analyse de l'échantillon qui s'écoule, pour obtenir des mesures cohérentes et précises. Lors d'un transport et/ou de vibrations éventuelles, l'alignement de la lumière a tendance à se dérégler, l'écoulement à ne pas se produire sous un volume constant et les graines à se compacter et ne plus se vider du dispositif. Or, jusqu'à présent aucune solution satisfaisante à ce problème n'a été trouvée.

Pour atteindre ce but, l'agencement selon l'invention est caractérisé en ce que qu'il comprend un mécanisme de réglage sélectif de la position du faisceau de lumière dans des plans vertical et horizontal et un dispositif de réglage sélectif pour assurer le parallélisme des rayons du faisceau de lumière.

De préférence, le dispositif d'obturation et d'ouverture comprend un cylindre rotatif dans le fond du réservoir du produit et qui comprend un alésage de passage du produit et en ce que l'alésage dans sa position d'ouverture forme un angle α prédéterminé par rapport à la direction horizontale de façon à constituer une rampe inclinée de glissement du produit s'écoulant dans la chambre de lecture, pour assurer l'homogénéité du produit dans la chambre.

Avantageusement, l'angle d'inclinaison α est spécifique pour différents types de produits.

Avantageusement, la quantité du produit s'écoulant dans la chambre de lecture est établie par le temps d'ouverture du dispositif d'obturation et d'ouverture.

En outre, le dispositif d'obturation est susceptible d'osciller autour de sa position d'ouverture d'un angle d'oscillation prédéterminé afin d'améliorer encore l'homogénéité du produit dans la chambre de lecture.

De préférence, le mécanisme de réglage de la position du faisceau de lumière comprend un dispositif porteur pour une source génératrice du faisceau de lumière, et comporte une pluralité de platines de réglage dont deux sont pivotantes autour d'axes respectivement vertical et horizontal à l'aide de vis actionnables de l'extérieur.

Avantageusement, une première platine est montée pivotante sur la platine de support autour d'un axe horizontal ou vertical sous l'effet de vis traversant la platine de support pour venir en appui par leurs extrémités internes sur la face en regard de la première platine, tandis que la seconde platine porteuse de la source de lumière est montée pivotante autour d'un axe vertical ou horizontal sur la première platine sous l'effet de vis traversant la platine de support et la première platine pour venir en appui contre la face en regard de la seconde platine.

Avantageusement, chaque platine est orientable par deux vis dont l'une sert de réglage et l'autre assure le maintien de la platine dans sa position réglée.

De préférence, les axes de platine sont formés chacun par deux vis pénétrant radialement dans la périphérie de la platine correspondante et qui servent, après le réglage, de moyens de blocage des platines dans leur position réglée.

Avantageusement, l'agencement comprend un bloc optique comprenant un organe de focalisation qui est déplaçable manuellement sur l'axe optique pour qu'une source génératrice du faisceau de lumière peut être positionnée dans le point focal de l'organe de focalisation.

Avantageusement, l'organe optique est vissé dans son support et axialement déplaçable par rotation manuelle.

L'invention sera mieux comprise, et d'autres buts, caractéristiques, détails et avantages de celle-ci apparaîtront plus clairement dans la description explicative qui va suivre faite en référence aux dessins schématiques annexés donnés uniquement à titre d'exemple illustrant un mode de réalisation de l'invention et dans lesquels :
- la figure 1 est une vue schématique qui illustre le principe d'un agencement de mesure spectrométrique de céréale ou analogue ;
- la figure 2 est une vue en perspective d'un agencement selon un mode de réalisation de l'invention ;
- la figure 3 est une autre vue en perspective de l'agencement selon un mode de réalisation de l'invention ;

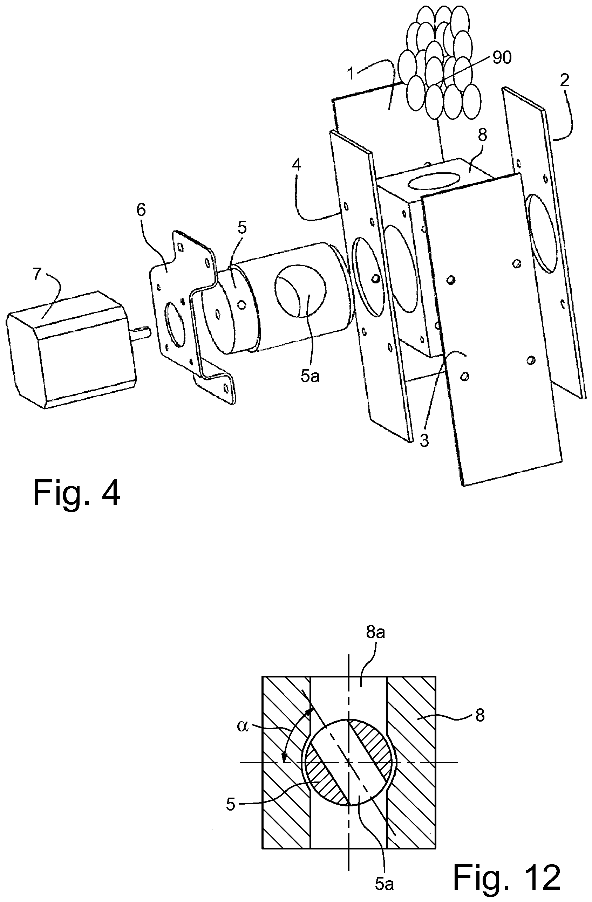

- la figure 4 est une vue en perspective et éclatée du sous-ensemble indiqué en I sur la figure 3 ;

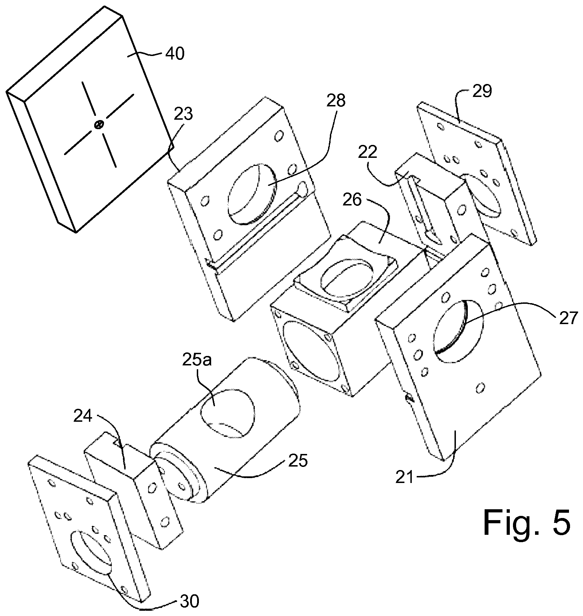

- la figure 5 est une vue en perspective et éclatée d'un second sous-ensemble selon l'invention indiqué en II sur la figure 3 ;

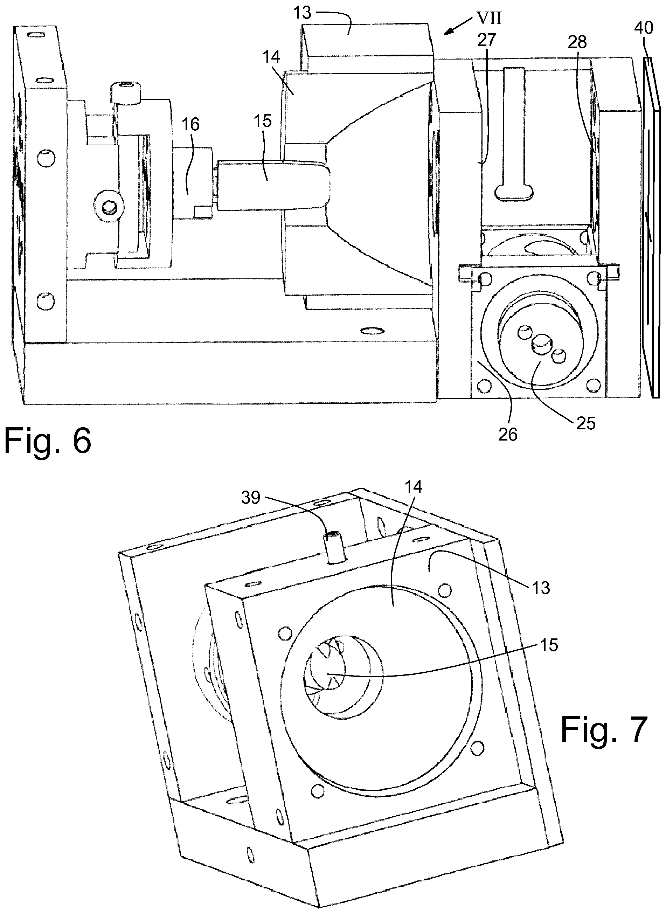

- la figure 6 est une vue montrant un troisième sous-ensemble de bloc optique selon un mode de réalisation de l'invention, indiqué en III de la figure 3 et accolé au sous-ensemble II ;
- la figure 7 est une vue en perspective et à plus grande échelle de la partie indiquée en VII sur la figure 6 ;

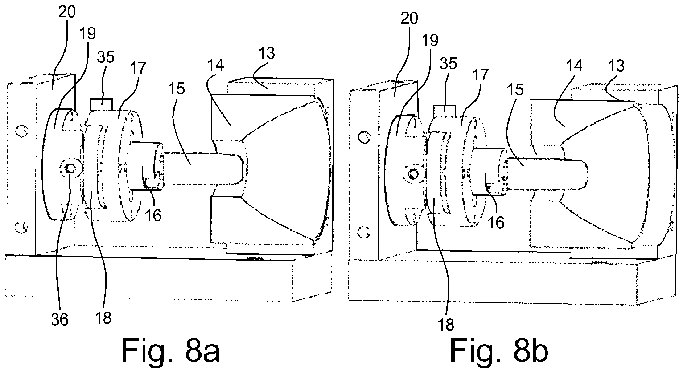

- les figures 8a et 8b sont deux vues en perspective du mécanisme de réglage de la lampe, selon un mode de réalisation de l'invention ;

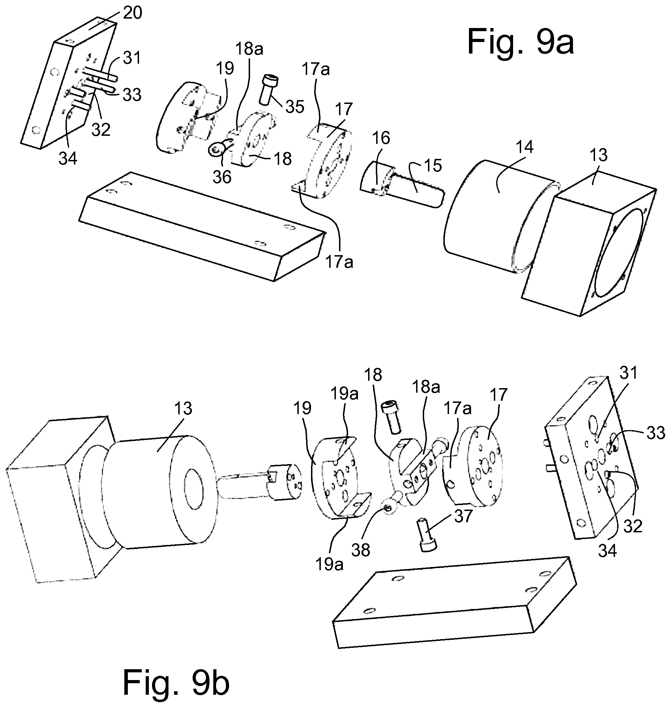

- les figures 9a et 9b sont deux vues en perspective et éclatées du mécanisme de réglage de la position de la lampe ;
- les figures 10a à 10d et 11a à 11d illustrent le procédé de positionnement de la source de lumière d'analyse dans l'axe optique ;
- la figure 12 est une vue schématique illustrant la disposition d'un mode de réalisation de l'invention pour assurer l'homogénéité de l'écoulement des grains dans la chambre de lecture et d'analyse.

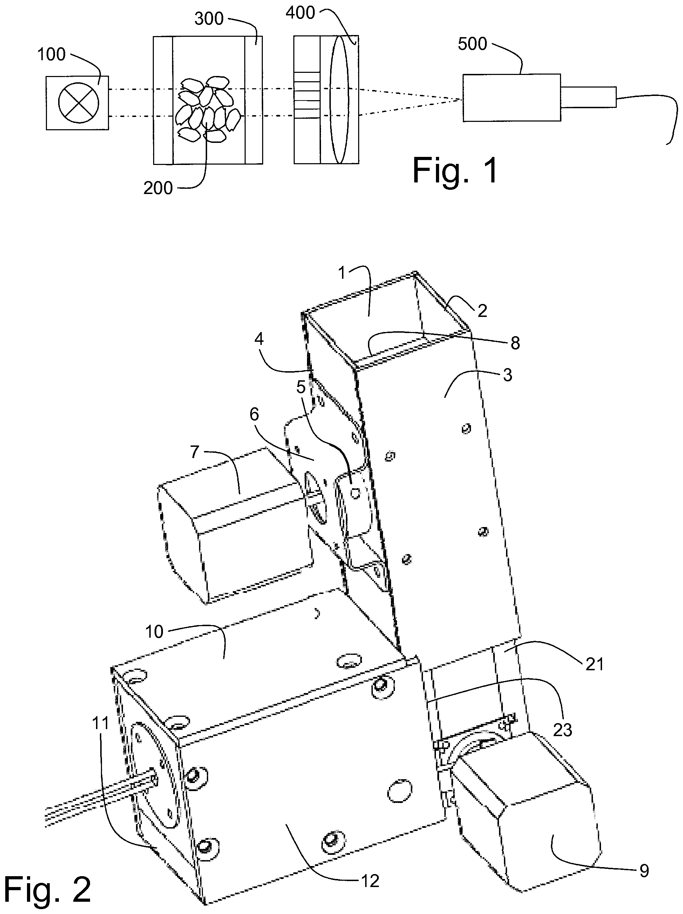

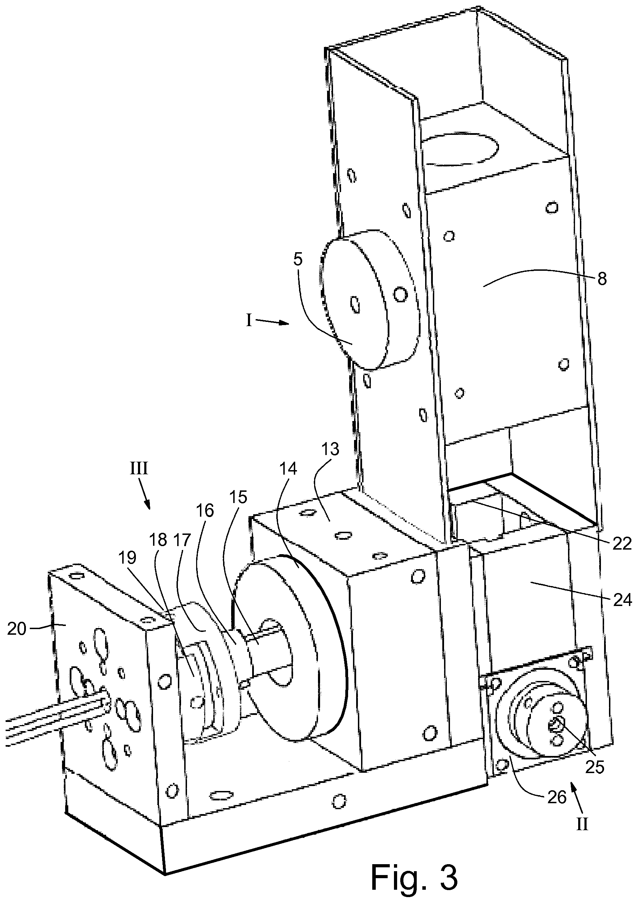

Comme le montre les figures 2 et 3, l'agencement de mesure spectrométrique de céréales oléagineux ou produits dérivés, selon un mode de réalisation de l'invention comprend trois sous-ensembles, à savoir un premier sous-ensemble I destiné à assurer l'écoulement des grains à analyser dans la chambre de lecture et d'analyse, un second ensemble II comportant la chambre de mesure et le dispositif d'évacuation des grains et un troisième sous-ensemble III qui constitue le bloc optique de l'agencement.

Le premier sous-ensemble I comprend un carter de forme parallélépipédique composé des parois 1, 2, 3 et 4, délimitant une chambre d'alimentation en grains comme cela est indiqué schématiquement, et en-dessous un corps de support 8 traversé axialement de haut en bas d'un alésage cylindrique 8a, et un cylindre d'obturation 5 disposé dans le support 8, perpendiculaire à l'axe de l'alésage 8a, pourvu d'un alésage cylindrique traversant 5a et rotatif entre une position dans laquelle l'alésage 5a permet le passage des grains dans la chambre de lecture et une position d'obturation de l'alésage 8a et donc de fermeture de la chambre d'alimentation. Le cylindre d'obturation 5 peut être actionné manuellement ou entrainé en rotation par un moteur 7 monté sur la paroi latérale 4 à l'aide d'une bride 6.

Le second sous-ensemble II comporte la chambre de lecture d'échantillon délimitée par un carter parallélépipédique composé des parois 21, 22, 23 et 24. La chambre de lecture est traversée par le faisceau de lumière de lecture et d'analyse. A cette fin, les parois 21 et 23 présentent des fenêtres 27, 28 de passage du faisceau. En dessous de la chambre est disposé un support cylindre 26 qui constitue le fond de la chambre de lecture. Dans ce support 26 est monté rotatif un cylindre d'obturation rotatif 25 pouvant être actionné manuellement ou entrainé en rotation par un moteur 9. Le cylindre d'obturation 25 est rotatif entre une position dans laquelle un perçage cylindrique 25a s'ouvre dans la chambre de lecture et permet l'évacuation des grains ayant été analysés, et une position angulaire dans laquelle le cylindre 25 obture le fond de la chambre du fait que le perçage soit orienté perpendiculairement.

Le troisième sous-ensemble III est un bloc optique comprenant un carter parallélépipédique formé par des parois 10, 11, 12 qui enferment un dispositif optique comportant un support 13 de réflecteur parabolique, un réflecteur parabolique 14 et une lampe 15 pourvue d'un culot 16 monté sur des platines d'ajustement 17, 18 et 19, ainsi qu'une plaque de support 20. De manière alternative, le dispositif optique comprend un support de lentille et une lentille en lieu et place du support 13 de réflecteur parabolique et du réflecteur parabolique 14. Les platines 17 et 18 sont orientables à l'aide de vis 31 à 34, vissées dans la plaque 20 qu'elles traversent et susceptibles d'être actionnées de l'extérieur. Ces vis traversent aussi la platine 19 pour pouvoir venir en appui dans leurs extrémités internes sur les platines 17 et 18, en actionnant ces vis il est possible de régler à l'aide de ces vis la position de la lampe 15 de la manière qui sera décrite plus loin.

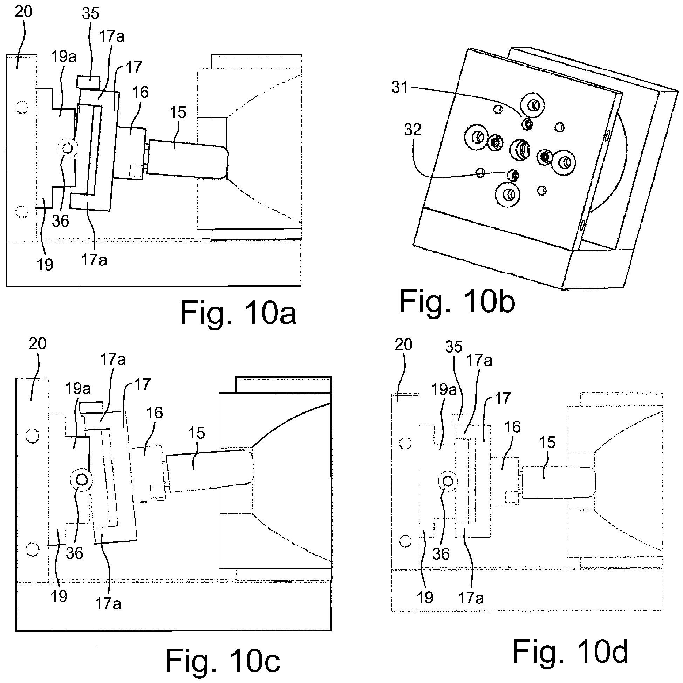

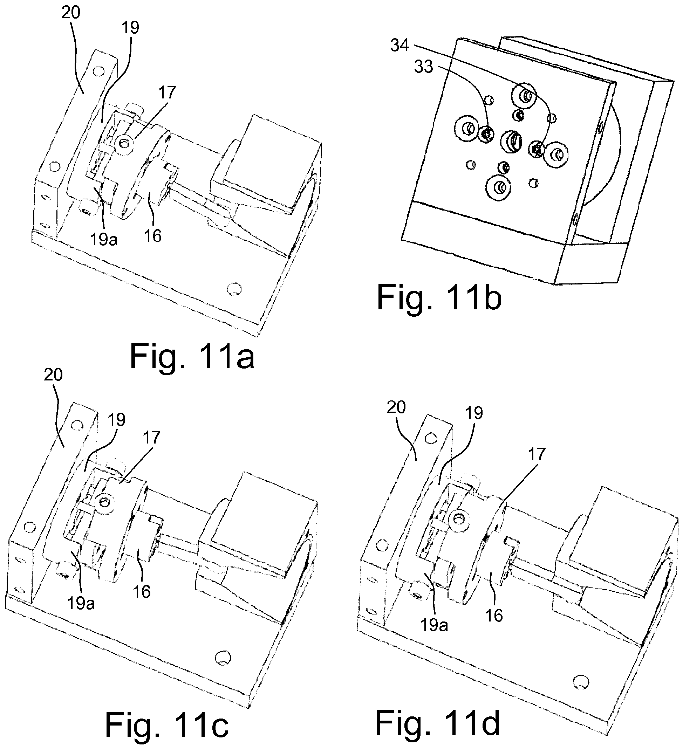

Il est à noter que la platine 19, comme on le voit sur les figures 10a à 10d et 11a-11d est en appui sur la face interne de la plaque 20. Elle comporte deux saillies 19a qui sont situées diamétralement opposées à la périphérie de la platine et traversées chacune par une vis 36 qui pénètre dans une nervure diamétrale 18a sur la face de la platine 18, qui est en regard de la platine 19. La platine 17 comporte à sa périphérie des saillies 17a également diamétralement opposées mais décalées des saillies 19a de la platine 19 d'un angle de 90°. Ces saillies 17a sont traversées par des vis 35 qui pénètrent dans la périphérie de la platine 18. Concernant la lampe 15, elle est fixée par son culot 16 sur la face avant libre de la platine 17. Les vis 35 et 36 constituent les axes de pivotement des platines 17 et 18 et permettent, après le réglage, de bloquer les platines dans leurs positions réglées.

Les platines 17, 18, 19 constituent le mécanisme de support et de réglage vertical et horizontal de la lampe 15, c'est à dire de la source de lumière, dans le cas d'espèce des filaments de la lampe. Ce mécanisme permet de positionner la source d'une façon simple dans l'axe optique du bloc optique. Le positionnement correct est vérifiable par exemple à l'aide d'un écran de projection indiqué schématiquement en 40 sur lequel une croix précise la position de l'axe optique.

Pour le réglage du faisceau afin que ces rayons soient parallèles, le réflecteur parabolique est mobile le long de l'axe optique. A cette fin le réflecteur est vissé dans son support. Ainsi la rotation du réflecteur permet son déplacement dans l'axe optique jusqu'à ce que la source, à savoir dan le cas présent, les filaments de la lampe, se trouve dans un point focal du réflecteur.

Concernant le positionnement de la source dans l'axe optique tout d'abord dans le plan vertical, on serre la vis 31 ou la vis 32 contre la face en regard de la platine 18, les vis 36 servant d'axes de pivotement, si la vis 31 est actionnée la vis 32 permet de bloquer la platine dans sa position réglée. Si c'est la vis 32 qui est actionnée, c'est la vis 31 qui maintient la platine bloquée dans sa position réglée.

Pour positionner le faisceau correctement dans le plan horizontal, on actionne soit la vis 33 soit la vis 34. Ces vis traversent la platine 18 pour venir en appui par leurs extrémités contre la face en regard de la platine 17. Les vis 38 constituent alors l'axe de pivotement vertical. Après le réglage des positions des platines 17 et 18, les vis qui constituent les axes de pivotement permettent le blocage des platines dans leur position réglée.

On constate que le mécanisme de positionnement du faisceau dans l'axe optique est très simple et facile à manipuler.

Selon une autre caractéristique avantageuse de l'invention, celle-ci permet d'assurer d'une manière très simple l'homogénéité des grains dans la chambre de mesure.

En effet, l'homogénéité est assurée grâce au cylindre d'obturation 5 qui est rotatif dans son support 8 pour obturer la chambre d'alimentation ou l'ouverture permettant l'écoulement des grains dans la chambre de lecture ou mesure. Or, dans sa position d'ouverture, l'alésage 5a n'est pas orienté verticalement, mais est seulement incliné par rapport à l'axe horizontal d'un angle prédéterminé. Ainsi, le paroi de l'alésage constituent une rampe de glissement des grains ce qui les singularise et évite leur agglomération.

L'angle d'inclinaison est spécifique pour les différents types de grains. Par exemple, pour le blé l'angle est de 60°, pour le colza seulement de 15°. La quantité des grains dans la chambre de mesure est également spécifique pour les différents types de grains et elle est déterminée par le temps d'ouverture.

Pour améliorer encore l'homogénéité des grains dans la chambre de mesure, on peut faire en sorte que le cylindre rotatif 5 oscille autour de son angle d'ouverture de quelques degrés prédéterminés, par exemple de deux degrés. En utilisant un moteur pas-à pas pour la rotation du cylindre, l'angle d'oscillation est facilement réalisable et réglable.

Bien entendu, dans le cadre des revendications annexées, de multiples modifications peuvent être apportées au mode de réalisation décrit et représenté à titre d'exemple non limitatif. Ainsi, au lieu d'utiliser un réflecteur parabolique, on pourrait aussi utiliser une lentille de focalisation appropriée.

## Revendications

1. Agencement de mesure spectrométrique de produits tels que des céréales, oléagineux ou produits dérivés, comprenant une chambre de lecture traversée par un faisceau de lumière de lecture et d'analyse et comportant l'échantillon du produit à analyser, un réservoir de produit à analyser, un dispositif **d'obturation et d'ouverture du réservoir du produit à analyser,** permettant au produit de s'écouler dans la chambre de lecture, déplaçable entre une position d'obturation du réservoir et une position d'ouverture permettant l'écoulement de produit dans la chambre de lecture, et un dispositif d'évacuation du produit de la chambre de lecture, **caractérisé en ce qu'**il comprend un mécanisme de réglage sélectif (17, 18, 19) de la position du faisceau de lumière dans des plans vertical et horizontal et un dispositif de réglage sélectif pour assurer le parallélisme des rayons du faisceau de lumière.

2. Agencement selon la revendication 1, **caractérisé en ce que** le dispositif d'obturation et d'ouverture comprend un cylindre (5) rotatif dans le fond du réservoir du produit et qui comprend un alésage (5a) de passage du produit et **en ce que** l'alésage dans sa position d'ouverture forme un angle α prédéterminé par rapport à la direction horizontale de façon à constituer une rampe inclinée de glissement du produit s'écoulant dans la chambre de lecture, pour assurer l'homogénéité du produit dans la chambre.

3. Agencement selon la revendication 2, **caractérisé en ce que** l'angle d'inclinaison α est spécifique pour différents types de produits.

4. Agencement selon la revendication 3, **caractérisé en ce que** la quantité du produit s'écoulant dans la chambre de lecture est établie par le temps d'ouverture du dispositif d'obturation et d'ouverture.

5. Agencement selon la revendication 2 ou 3, **caractérisé en ce que** le dispositif d'obturation est susceptible d'osciller autour de sa position d'ouverture d'un angle d'oscillation prédéterminé afin d'améliorer encore l'homogénéité du produit dans la chambre de lecture.

6. Agencement selon l'une des revendications 1 à 5, **caractérisé en ce que** le mécanisme de réglage de la position du faisceau de lumière comprend un dispositif porteur pour une source (15) génératrice du faisceau de lumière, et comporte une pluralité de platines de réglage (17, 18, 19) dont deux (17, 18) sont pivotantes autour d'axes respectivement vertical et horizontal à l'aide de vis (31 à 34) actionnables de l'extérieur.

7. Agencement selon la revendication 6, **caractérisé en ce qu'**une première platine (18) est montée pivotante sur la platine de support (19) autour d'un axe horizontal ou vertical sous l'effet de vis traversant la platine de support (19) pour venir en appui par leurs extrémités internes sur la face en regard de la première platine (18), tandis que la seconde platine (17) porteuse de la source de lumière est montée pivotante autour d'un axe vertical ou horizontal sur la première platine (18) sous l'effet de vis traversant la platine de support (19) et la première platine (18) pour venir en appui contre la face en regard de la seconde platine (17).

8. Agencement selon la revendication 7, **caractérisé en ce que** chaque platine (17, 18) est orientable par deux vis (31 à 34), dont l'une sert de réglage et l'autre assure le maintien de la platine dans sa position réglée.

9. Agencement selon l'une des revendications 6 à 8, **caractérisé en ce que** les axes de platine sont formés chacun par deux vis pénétrant radialement dans la périphérie de la platine correspondante et qui servent, après le réglage, de moyens de blocage des platines dans leur position réglée.

10. Agencement selon l'une des revendications 1 à 9, **caractérisé en ce qu'**il comprend un bloc optique comprenant un organe de focalisation qui est déplaçable manuellement sur l'axe optique pour qu'une source (15) génératrice du faisceau de lumière peut être positionnée dans le point focal de l'organe de focalisation.

11. Agencement selon la revendication 10, **caractérisé en ce que** l'organe optique est vissé dans son support et axialement déplaçable par rotation manuelle.

## Patentansprüche

1. Anordnung zur spektrometrischen Messung von Produkten wie Getreide, Ölfrüchte oder Derivate, umfassend eine Ablesekammer, die von einem Ablese- und Analyselichtbündel durchquert wird und eine Probe des zu analysierenden Produkts aufweist, einen Vorratsbehälter mit zu analysierendem Produkt, eine Verschluss- und Öffnungsvorrichtung des Vorratsbehälters des zu analysierenden Produkts, die es dem Produkt erlaubt, in die Ablesekammer zu strömen, die zwischen einer verschlossenen Position des Vorratsbehälters und einer geöffneten Position, welche das Strömen von Produkt in die Ablesekammer erlaubt, verlagerbar ist, und eine Vorrichtung zum Entfernen des Produkts aus der Ablesekammer, **dadurch gekennzeichnet, dass** sie einen Mechanismus zum selektiven Einstellen (17, 18, 19) der Position des Lichtbündels in vertikaler und horizontaler Ebene und eine Vorrichtung zum selektiven Einstellen, um die Parallelität der Strahlen des Lichtbündels zu sichern, umfasst.

2. Anordnung nach Anspruch 1, **dadurch gekennzeichnet, dass** die Verschluss- und Öffnungsvorrichtung einen rotierenden Zylinder (5) im Boden des Vorratsbehälters des Produkts umfasst und der eine Bohrung (5a) für den Durchgang des Produkts aufweist und dadurch, dass die Bohrung in ihrer geöffneten Position einen in Bezug auf die horizontale Richtung vorher festgelegten Winkel α bildet, um eine geneigte Rampe für das Gleiten des in die Ablesekammer strömenden Produkts zu bilden, um die Homogenität des Produkts in der Kammer zu sichern.

3. Anordnung nach Anspruch 2, **dadurch gekennzeichnet, dass** der Neigungswinkel α für unterschiedliche Produkttypen spezifisch ist.

4. Anordnung nach Anspruch 3, **dadurch gekennzeichnet, dass** die Menge des Produkts, das in die Ablesekammer strömt, von der Öffnungszeit der Verschluss- und Öffnungsvorrichtung festgelegt wird.

5. Anordnung nach Anspruch 2 oder 3, **dadurch gekennzeichnet, dass** die Verschlussvorrichtung imstande ist, um um ihrer geöffnete Positionnierung gemäß einem vorher festgelegten Schwingungswinkel zu schwingen, um die Homogenität des Produkts in der Ablesekammer weiter zu verbessern.

6. Anordnung nach einem der Ansprüche 1 bis 5, **dadurch gekennzeichnet, dass** der Mechanismus zum Einstellen der Position des Lichtbündels eine Trägervorrichtung für eine das Lichtbündel erzeugende Quelle (15) umfasst und eine Vielzahl von Einstellplatten (17, 18, 19) aufweist, von denen zwei (17, 18) mit Hilfe von außen bedienbaren Schauben (31 bis 34) um eine jeweilige vertikale und horizontale Achse schwenkbar sind.

7. Anordnung nach Anspruch 6, **dadurch gekennzeichnet, dass** eine erste Platte (18) auf der Stützplatte (19) um eine horizontale oder vertikale Achse unter der Wirkung einer Schraube schwenkend angebracht ist, die die Stützplatte (19) durchquert, um sich mit ihren inneren Enden auf der Fläche gegenüber der ersten Platte (18) abzustützen, wogegen die zweite Trägerplatte (17) der Lichtquelle um eine vertikale oder horizontale Achse auf der ersten Platte (18) unter der Wirkung einer Schraube schwenkend angebracht ist, die die Stützplatte (19) und die erste Platte (18) durchquert, um sich auf der Fläche gegenüber der zweiten Platte (17) abzustützen.

8. Anordnung nach Anspruch 7, **dadurch gekennzeichnet, dass** jede Platte (17, 18) mit zwei Schrauben (31 bis 34) ausrichtbar ist, von denen eine zum Einstellen dient und die andere den Halt der Platte in ihrer eingestellten Position sichert.

9. Anordnung nach einem der Ansprüche 6 bis 8, **dadurch gekennzeichnet, dass** die Plattenachsen jeweils von zwei Schrauben gebildet sind, die radial in den Umfang der entsprechenden Platte eindringen und die nach dem Einstellen als Blockiermittel der Platten in ihrer eingestellten Position dienen.

10. Anordnung nach einem der Ansprüche 1 bis 9, **dadurch gekennzeichnet, dass** sie einen optischen Block umfasst, der ein Fokussierorgan umfasst, das manuell auf der optischen Achse verlagerbar ist, damit eine Quelle (15), welche das Lichtbündel erzeugt, im Fokalpunkt des Fokussierorgans positionierbar ist.

11. Anordnung nach Anspruch 10, **dadurch gekennzeichnet, dass** das optische Organ in ihren Halter geschraubt ist und durch manuelle Rotation axial verlagerbar ist.

## Claims

1. An arrangement for the spectrometric measurement of products such as cereals, oleaginous products or derived products, comprising a reading chamber passed through by a beam of light for reading and analysis and including the sample of the product to be analyzed, a reservoir for product to be analyzed, a device for **closing and opening the reservoir of the product to be analyzed,** allowing the product to flow into the reading chamber, movable between a position closing the reservoir and an open position allowing the flow of product into the reading chamber, and a device for discharging the product from the reading chamber, **characterized in that** it comprises a mechanism for selectively adjusting (17, 18, 19) the position of the beam of light in vertical and horizontal planes and a selective adjustment device for providing the parallelism of the rays of the beam of light.

2. The arrangement according to claim 1, **characterized in that** the closing and opening device comprises a rotary cylinder (5) in the bottom of the reservoir for the product and which comprises a bore (5a) for passage of the product and **in that** the bore, in its open position, forms a predetermined angle α relative to the horizontal direction so as to form an inclined ramp for sliding of the product flowing in the reading chamber, to provide the homogeneity of the product in the chamber.

3. The arrangement according to claim 2, **characterized in that** the incline angle α is specific for different types of products.

4. The arrangement according to claim 3, **characterized in that** the quantity of product flowing in the reading chamber is established by the opening time of the closing and opening device.

5. The arrangement according to claim 2 or 3, **characterized in that** the closing device is able to oscillate around its open position by a predetermined oscillating angle in order to further improve the homogeneity of the product in the reading chamber.

6. The arrangement according to one of claims 1 to 5, **characterized in that** the mechanism for adjusting the position of the beam of light comprises a carrier device for a source (15) **generating the beam of light,** and includes a plurality of adjusting platens (17, 18, 19), two of which (17, 18) pivot around respective vertical and horizontal axes using screws (31 to 34) able to be actuated from the outside.

7. The arrangement according to claim 6, **characterized in that** a first platen (18) is mounted pivoting on the support platen (19) around a horizontal or vertical axis under the effect of screws passing through the support platen (19) to bear by their inner ends on the opposite face of the first platen (18), while the second platen (17) carrying the light source is mounted pivoting around a vertical or horizontal axis on the first plate (18) under the effect of screws passing through the support platen (19) and the first platen (18) to bear against the opposite face of the second platen (17).

8. The arrangement according to claim 7, **characterized in that** each platen (17, 18) is able to be oriented by two screws (31 to 34), one of which is used for adjustment and the other of which keeps the platen in its adjusted position.

9. The arrangement according to one of claims 6 to 8, **characterized in that** the platen axes are each formed by two screws radially penetrating the periphery of the corresponding platen and which serve, after adjustment, as means for locking the platens in their adjusted position.

10. The arrangement according to one of claims 1 to 9, **characterized in that** it comprises an optical unit comprising a focusing member that is movable manually on the optical axis so that a source (15) generating the beam of light can be positioned in the focal point of the focusing member.

11. The arrangement according to claim 10, **characterized in that** the optical member is screwed in its support and axially movable by manual rotation.
